(19) Europäisches Patentamt
European Patent Office
Office européen des brevets

(11) **EP 4 495 233 A1**

(12) **EUROPEAN PATENT APPLICATION**
published in accordance with Art. 153(4) EPC

(43) Date of publication:
**22.01.2025 Bulletin 2025/04**

(51) International Patent Classification (IPC):
*C12N 9/38* (2006.01)    *A23C 9/152* (2006.01)
*C12N 9/58* (2006.01)    *C12P 1/00* (2006.01)

(21) Application number: 23770923.3

(22) Date of filing: **17.03.2023**

(52) Cooperative Patent Classification (CPC):
**A23C 9/152; C12N 9/2468; C12N 9/58; C12P 1/00**

(86) International application number:
**PCT/JP2023/010684**

(87) International publication number:
**WO 2023/176971 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.03.2022   JP 2022044171**

(71) Applicant: **Godo Shusei Co., Ltd.**
**Matsudo-shi, Chiba 271-0064 (JP)**

(72) Inventors:
• **NIIKAWA, Yuki**
**Matsudo-shi, Chiba 271-0064 (JP)**
• **MIYAUCHI, Kiyomi**
**Matsudo-shi, Chiba 271-0064 (JP)**
• **AOKI, Shohei**
**Matsudo-shi, Chiba 271-0064 (JP)**
• **BABA, Masahiro**
**Matsudo-shi, Chiba 271-0064 (JP)**
• **YUGA, Masaki**
**Matsudo-shi, Chiba 271-0064 (JP)**

(74) Representative: **Hoffmann Eitle**
**Patent- und Rechtsanwälte PartmbB**
**Arabellastraße 30**
**81925 München (DE)**

(54) **ENZYME-CONTAINING SOLUTION AND METHOD FOR PRODUCING DAIRY PRODUCTS**

(57)    An object of the present invention is to provide a method for producing a dairy product using an enzyme-containing solution that suppresses generation of coagulum or curd in a dairy product. The enzyme-containing solution contains acidic lactase and has an acidic protease activity of 100 U/g or less. The enzyme-containing solution has a ratio of the acidic protease activity (U/g) to an acidic lactase activity (U/g) of 0.01 or less. The method for producing a dairy product uses the enzyme-containing solution having the acidic protease activity to casein of 300 mU/g or less.

**FIG. 1**

## Description

Technical Field

The present invention relates to an enzyme-containing solution and a method for producing a dairy product.

Background Art

Lactose is present in milk and a dairy product. In many humans, lactose in a dairy product is degraded into glucose and galactose by lactase present in the small intestine. However, in some humans, the amount of lactase present in the small intestine is small, lactose cannot be sufficiently degraded, and a symptom of diarrhea or indigestion occurs. In order to suppress this symptom, lactase is widely used in a food industry and a pharmaceutical industry.

In general, lactase used in foods, pharmaceuticals, and the like is derived from microorganisms, and is obtained from a culture solution in which microorganisms such as yeast (Kluyveromyces lactis and Kluuyveromyces fragilis), mold (Aspergillus oryzae and Penicillum multicolor), and bacteria (Bacillus circulans) are cultured. Lactase varies in reaction pH, lactose-degrading ability, oligosaccharide-producing ability, and the like depending on a microorganism species from which lactase is derived, and is selected according to a purpose of use and a reaction condition (Non Patent Literature 1). Mold-derived lactase is characterized by acid resistance, and is also called acidic lactase because lactase hydrolyzes lactose under a low pH intragastric environment, and is mainly used in pharmaceutical applications (Non Patent Literature 2).

Citation List

Patent Literature

Non Patent Literature 1: Milk Science Vol. 60, No.2 2011
Non Patent Literature 2: Milk Science Vol. 61, No.3 2012

Summary of Invention

Technical Problem

Aspergillus oryzae, which is a type of mold, produces acidic lactase. When this acidic lactase was allowed to act on a raw material milk for a longer time than usual, coagulum was generated in the milk (dairy product) after the action, or the whole milk became curd in a severe case.

An object of the present invention is to provide an enzyme-containing solution that suppresses generation of coagulum or curd in a dairy product.

An object of the present invention is to provide a method for producing a dairy product using an enzyme-containing solution that suppresses generation of coagulum or curd in a dairy product.

Solution to Problem

The present invention has solved the problem of the present invention by having the following technical configurations.

(1) An enzyme-containing solution containing acidic lactase and having an acidic protease activity of 100 U/g or less.
(2) The enzyme-containing solution according to (1), in which a ratio of the acidic protease activity (U/g) to the acidic lactase activity (U/g) is 0.01 or less.
(3) The enzyme-containing solution according to (1) or (2), in which a ratio of a carboxymethyl cellulase activity (U/g) to the acidic lactase activity (U/g) is $0.3 \times 10^{-4}$ or less.
(4) A method for producing a dairy product, including a step of adding an enzyme-containing solution to a raw material milk containing casein, in which the enzyme-containing solution contains acidic lactase and has an acidic protease activity of 100 U/g or less.
(5) The method for producing a dairy product according to (4), in which a content of the casein in a raw material milk is 3 to 20% by mass.
(6) The method for producing a dairy product according to (4) or (5), in which the raw material milk is an acidic milk.

(7) The method for producing a dairy product according to any one of (4) to (6), in which the acidic protease activity to the casein is 300 mU/g or less.

Advantageous Effects of Invention

According to the invention of the enzyme-containing solution and the method for producing a dairy product of the present invention, it is possible to provide an enzyme-containing solution that reduces the amount of lactose without generating curd even when acidic lactase is allowed to act on milk for a long time.

Brief Description of Drawings

Fig. 1 is a graph illustrating temporal changes in acidic protease and an acidic lactase residual ratio during heat treatment at 50°C at pH 6.5.
Fig. 2 is a diagram illustrating a state of an acidic milk when an acidic lactase-containing solution is allowed to act on an acidic milk containing 35% of a milk component at 33°C for twelve weeks.
Fig. 3 is a diagram illustrating a state of an acidic milk when an acidic lactase-containing solution is allowed to act on an acidic milk containing 25% of a milk component at 33°C for twelve weeks.

Description of Embodiments

<Enzyme-containing solution>

An enzyme-containing solution of the present invention is characterized by containing acidic lactase and having an acidic protease activity of 100 U/g or less. This makes it possible to provide an enzyme-containing solution that suppresses generation of coagulum or curd in a dairy product while reducing the amount of lactose contained in the dairy product.

[Acidic lactase]

In the present invention, lactase having a lactose-degrading action within a pH range of 3.0 to 5.0 is referred to as acidic lactase. The lactose-degrading action is preferably exhibited within a pH range of 3.5 to 4.5.

(Derivation of acidic lactase)

Examples of microorganisms that produce acidic lactase include mold such as the genus Aspergillus or the genus Penicillium, and bacteria such as Bifidobacterium. Any of these may be used in the present invention.
As the genus Aspergillus, for example, Aspergillus niger, Aspergillus kawachii, and Aspergillus oryzae can be used. As the genus Penicillium, for example, Penicillium multicolor can be used. As the genus Bifidobacterium, for example, Bifidobacterium bifidum and Bifidobacterium breve can be used.
In the present invention, it is preferable to use a microorganism that secretes acidic lactase extracellularly. Examples of a microorganism corresponding thereto include Aspergillus niger, Aspergillus kawachii, Aspergillus oryzae, and Penicillium multicolor.
In the present invention, it is preferable to use Aspergillus that produces a large amount of protein. A production amount of acidic lactase can be increased by continuously breeding strains without using a genetic recombination technique.

(Acidic lactase activity)

An acidic lactase activity contained in the enzyme-containing solution of the present invention is preferably in a range of 10 U/g to 100,000 U/g, more preferably in a range of 100 U/g to 50,000 U/g, and still more preferably in a range of 500 U/g to 10,000 U/g by a measurement method described later.

[Acidic protease]

The acidic protease in the present invention does not refer to a specific protein, but is the sum of proteins that act as acidic protease. The acidic protease can be measured by a measurement method described later.

(Acidic protease activity)

The acidic protease activity (U/g) contained in the enzyme-containing liquid of the present invention is preferably less than 100. The acidic protease activity (U/g) may be 50 U/g or less, 40 U/g or less, 30 U/g or less, 20 U/g or less, 10 U/g or less, or a detection limit or less. It is preferable to lower the acidic protease activity from a viewpoint of suppressing generation of coagulum or curd in a dairy product.

When a genetically modified microorganism is used, it is easy to efficiently purify and obtain only acidic lactase. Therefore, the acidic protease is preferably the detection limit or less.

When a non-genetically modified microorganism is used, it is technically very difficult to completely separate acidic lactase and acidic protease from each other. For example, Aspergillus oryzae has 100 or more types of protease genes on a genome, and 20 or more types of the protease genes are acidic protease genes. When Aspergillus oryzae is cultured to obtain acidic lactase, not only acidic lactase but also acidic protease is usually present in a culture solution or a culture. Complete removal of the acidic protease is not economical because a yield of the acidic lactase is lowered and purification cost is also increased. Therefore, the acidic protease activity contained in the enzyme-containing liquid of the present invention is determined within the above range in consideration of a dairy product to be used, an enzyme reaction time, and temperature. When a non-genetically modified microorganism is used, a lower limit value of the acidic protease activity contained in the enzyme-containing solution is, for example, 500 mU/g.

[Activity ratio (protease activity/lactase activity)]

By setting a ratio of the acidic protease activity (U/g) to the acidic lactase activity (U/g) contained in the enzyme-containing solution of the present invention to a certain value or less, it is easy to provide an enzyme-containing solution that suppresses generation of coagulum or curd in a dairy product.

When a genetically modified microorganism is used, the ratio of the acidic protease activity (U/g) to the acidic lactase activity (U/g) is preferably zero. This is because when a genetically modified microorganism is used, it is relatively easy to set the acidic protease activity contained in the enzyme-containing solution to zero.

When a non-genetically modified microorganism is used, the ratio of the acidic protease activity (U/g) to the acidic lactase activity (U/g) is preferably 0.01 or less, and more preferably 0.005 or less. When a non-genetically modified microorganism is used, it is very difficult to set the acidic protease activity contained in the enzyme-containing solution to zero. When a non-genetically modified microorganism is used, a lower limit value of the acidic protease to the acidic lactase activity contained in the enzyme-containing solution is, for example, $0.5 \times 10^{-4}$.

[Carboxymethyl cellulase activity]

A carboxymethyl cellulase activity (U/g) contained in the enzyme-containing solution of the present invention is preferably 0.35 U/g or less, more preferably 0.25 U/g or less, and still more preferably 0.10 U/g or less by a measurement method described later. A lower limit value of the carboxymethyl cellulase activity contained in the enzyme-containing solution is, for example, $7.5 \times 10^{-6}$ U/g.

This can reduce degradation of a cellulose-based substance when the cellulose-based substance is contained in a raw material milk or a dairy product.

(Activity ratio (carboxymethyl cellulase activity/acidic lactase activity))

A ratio of the carboxymethyl cellulase activity (U/g) to the acidic lactase activity (U/g) contained in the enzyme-containing solution of the present invention is preferably $0.3 \times 10^{-4}$ or less. This can reduce degradation of a cellulose-based substance when the cellulose-based substance is contained in a raw material milk or a dairy product even if the enzyme-containing solution of the present invention is used.

[Stabilizer]

The amount of a stabilizer contained in the enzyme-containing solution is preferably 10% by mass to 90% by mass, more preferably 20% by mass to 80% by mass, still more preferably 30% by mass to 70% by mass, and particularly preferably 40% by mass to 60% by mass. When the amount of the stabilizer is the lower limit value or more, it is easy to maintain the lactase activity in the enzyme-containing solution for a long period of time. When the amount of the stabilizer is more than the upper limit value, viscosity of the lactase solution increases, and therefore a filtration time is prolonged and workability is deteriorated.

Examples of the stabilizer include glycerin and sorbitol.

[Optional component]

The enzyme-containing solution of the present invention may contain various components as necessary. Specific examples thereof include metal salts that contribute to stabilization of lactase and protease, various saccharides, ascorbic acid and the like, starch as an excipient for improving usability, dextrin, and inorganic salts having a buffering action.

[pH of enzyme-containing solution]

The enzyme-containing solution of the present invention preferably has pH of 3.0 to 7.0. A lower limit value of the pH may be 3.5 or more or 4.0 or more. An upper limit value of the pH may be 6.5 or less, 6.0 or less, 5.5 or less, or 5.0 or less. These lower limit values and upper limit values can be appropriately combined.
When the pH of the enzyme-containing solution is the lower limit value or more, it is possible to suppress a phenomenon that the acidic lactase activity is more easily reduced as a storage period increases.
When the pH of the enzyme-containing solution is the upper limit value or less, it is possible to suppress a phenomenon that the acidic lactase activity is more easily reduced as a storage period increases.

<Method for producing dairy product>

The present invention is a method for producing a dairy product, including a step of adding an enzyme-containing solution to a raw material milk containing casein, in which the enzyme-containing solution contains acidic lactase and has an acidic protease activity of 100 U/g or less.
This makes it possible to provide a method for producing a dairy product using an enzyme-containing solution that suppresses generation of coagulum or curd in a dairy product.

[Raw material milk and dairy product]

In the present invention, a milk before addition of the enzyme-containing solution is referred to as a raw material milk, and a milk after the addition of the enzyme-containing solution (including a milk during enzyme reaction) is referred to as a dairy product.
The raw material milk is not limited as long as it contains casein. Examples of a material constituting the raw material milk include water, raw milk, sterilized milk, skimmed milk, whole fat powder, skimmed milk powder, butter milk, butter, cream, a whey protein concentrate (WPC), a whey protein isolate (WPI), a whey protein hydrolysate (WPH), casein protein, milk protein, $\alpha$ (alpha)-La, $\beta$ (beta)-Lg, fermented milk, a thickener, and a gelling agent and so on. A plurality of these materials may be used in combination.
The solid content contained in the raw material milk or the dairy product is preferably 10 to 50% by mass, more preferably 15 to 45% by mass, still more preferably 20 to 40% by mass, and particularly preferably 25 to 35% by mass.

(Casein)

When casein contained in the raw material milk reacts with acidic protease contained in the enzyme-containing solution, coagulum or curd is generated in the dairy product. The amount of casein contained in the raw material milk is important. The type of casein is not limited. For example, the amount of casein may be a sum of $\alpha$ casein, $\beta$ casein, $\kappa$ casein, and the like. A ratio among these is not limited. Intact casein that has not been physically damaged is more preferable.
The amount of casein contained in commercially available milk is about 3% by mass. A raw material milk or a dairy product that can be preferably used in the present invention is characterized in that the amount of casein contained in the raw material milk or the dairy product is larger than the amount of casein contained in commercially available milk. The amount of casein contained in the raw material milk or the dairy product in the present invention is preferably 3 to 25% by mass, more preferably 3 to 20% by mass, and still more preferably 3 to 15% by mass of the raw material milk. A lower limit value of the amount of casein contained in the raw material milk or the dairy product may be 5% by mass, 7% by mass or more, or 10% by mass or more. These lower limit values and upper limit values can be appropriately combined.
When the amount of casein contained in the raw material milk is less than the lower limit value, there is no possibility that coagulum or curd is generated in the dairy product even by adding the enzyme-containing solution.
When the amount of casein contained in the raw material milk is the upper limit value or less, it is possible to suppress generation of coagulum or curd in the dairy product.

(pH)

The pH of the raw material milk and the dairy product are important for generation of coagulum or curd in the dairy product. An isoelectric point of casein is pH 4.6, and therefore casein is likely to precipitate around this pH. The present invention makes it difficult for casein to precipitate even in a range close to the isoelectric point of casein, that is, under a condition where casein is likely to precipitate. This can be achieved by setting the acidic protease in the enzyme-containing solution of the present invention to 5 to 100 U/g or less as described above.

The pH of the raw material milk of the present invention is 3.0 to 5.0, and preferably 3.5 to 4.5. It is preferable to use an acidic milk as the raw material milk. The pH of the acidic milk is 3.0 to 5.0, and preferably 3.5 to 4.5.

The pH of the dairy product of the present invention is 3.0 to 5.0, and preferably 3.5 to 4.5. The dairy product is preferably in a liquid form.

[Final enzyme concentration in dairy product]

As for concentrations of the acidic lactase and the acidic protease in the dairy product in the method for producing a dairy product of the present invention, the concentration of the acidic lactase is preferably in a range of 10 to 5,000 U/L, more preferably in a range of 100 to 4,000 U/L, and still more preferably 500 to 3,000 U/L. The concentration of the acidic protease is preferably 100 U/L or less. The concentration may be 50 U/L or less, 40 U/L or less, 30 U/L or less, 20 U/L or less, 10 U/L or less, or a detection limit or less.

[Reaction condition of enzyme]

As a reaction condition of an enzyme in the method for producing a dairy product of the present invention, a concentration of lactose in the dairy product is preferably 50% or less, and may be 40% or less, 30% or less, 20% or less, 10% or less, 5% or less, 1% or less, or 0.1% or less with respect to 100% of lactose in the raw material milk. A lower limit value of the concentration of lactose may be 0%.

A reaction temperature of the enzyme in the method for producing a dairy product of the present invention is preferably in a range of 1°C to 40°C, preferably in a range of 2°C to 30°C, and more preferably in a range of 5°C to 25°C.

A reaction time of the enzyme in the method for producing a dairy product of the present invention is preferably one hour or more, and may be two hours or more, three hours or more, five hours or more, ten hours or more, twelve hours or more, 24 hours or more, two days or more, or three days or more. As long as an enzyme reaction proceeds under a sterile condition, an upper limit of the reaction time of the enzyme may be, for example, one year or less, and may be 180 days or less, 120 days or less, 90 days or less, 60 days or less, 30 days or less, 15 days or less, or seven days or less. It is also a characteristic of the present invention that it is assumed that the reaction time of the enzyme is long as described above, and for this purpose, the acidic protease activity needs to be a predetermined value or less. When the reaction time of the enzyme is long, it may be considered that the enzyme reaction proceeds in a product. Among the enzyme reactions, a lactase reaction does not further proceed when lactose and a degradation product reach an equilibrium state. Among the enzyme reactions, a protease reaction may be considered to continue over a longer period of time than the lactase reaction because a raw material milk and a dairy product each having a casein content higher than usual are used in the present invention.

[Acidic protease activity to casein]

An acidic protease activity to casein is preferably 300 mU/g or less, and may be 250 mU/g or less, 200 mU/g or less, 150 mU/g or less, 100 mU/g or less, or 50 mU/g or less. A lower limit value thereof may be 0 mU/g.

When the acidic protease activity to casein is the upper limit value or less, it is possible to suppress a problem that coagulum is generated in the dairy product or curd is generated in the entire milk.

Hereinafter, the present invention will be described with reference to Examples, but the present invention is not limited thereto.

<Evaluation method>

(Method for measuring acidic lactase activity)

A method for measuring the acidic lactase activity is, for example, as follows. The acidic lactase activity was measured by hydrolysis of a substrate o-nitrophenyl-β-galactopyranoside (ONPG) to o-nitrophenyl and galactose. The reaction is terminated by addition of sodium carbonate. The o-nitrophenyl formed turns yellow in an alkaline medium, and a change in absorbance is used to measure an enzyme activity (expressed in U/g). This procedure is described in the United States Food Chemicals Codex (FCC) 4th edition, July 1, 1996, page 802 to 803/lactase (acidic) (β-galactosidase) activity.

(Method for measuring acidic protease activity)

A method for activating the acidic protease is, for example, as follows. The acidic protease activity was measured using release of tyrosine from a substrate casein as an index. For a buffer solution for diluting the enzyme-containing solution, a 0.05 M glycine-hydrochloric acid buffer solution (pH 3.5) was used. 3.75 g of glycine was dissolved in 700 mL of distilled water, the pH was adjusted to 3.5 with 1 N hydrochloric acid, and then a volume thereof was adjusted to 1 L with distilled water.

For a precipitation reagent, 18.0 g of trichloroacetic acid, 18.0 g of anhydrous sodium acetate, and 19.8 g of glacial acetic acid were dissolved in about 600 mL of purified water, pH thereof was adjusted to 4.0, and then a volume thereof was adjusted to 1 L with purified water. For a substrate solution, 1.15 g of milk casein (Product No. 218682 manufactured by CalbioChem Ltd.) is weighed, and 80 mL of purified water and 1.28 mL of 1 N hydrochloric acid are added thereto in a 200 mL beaker and stirred. The resulting solution is heated in a boiling water bath for 30 minutes while being occasionally stirred. The solution is cooled with water, and then 0.6 g of glycine is added thereto and dissolved therein. pH of the solution is adjusted to 3.5 with 1 N sodium hydroxide, and a volume thereof is adjusted to 200 mL with purified water. The solution is prepared before use, and used within 24 hours in refrigerator storage.

For a calibration curve, 0 mL, 1 mL (0.2 mg), 2 mL (0.4 mg), 3 mL (0.6 mg), 4 mL (0.8 mg), or 5 mL (1.0 mg) of an L-tyrosine standard stock solution is dispensed into a test tube, and a 0.05 M glycine-hydrochloric acid buffer (pH 3.5) is added thereto such that a total volume thereof is 6 mL. 5 mL of a precipitation reagent is added thereto to obtain a standard solution. An absorbance of the standard solution having each concentration at 275 nm is measured to prepare the calibration curve, and a factor (F) is calculated.

In activity measurement, 0.5 mL of a sample solution (enzyme-containing solution) is put in a test tube and heated at 37°C for five minutes, then 2.5 mL of the substrate solution that has been heated in advance is added thereto and mixed, and the mixture is caused to react at 37°C for 30 minutes. 2.5 mL of a precipitation reagent was added thereto and mixed, and the mixture was allowed to stand at 37°C for 30 minutes. The mixture was allowed to stand in an ice-water bath for about five minutes and then filtered with No. 4A filter paper (manufactured by Advantec), and an absorbance of the filtrate was measured at 275 nm. A blank was prepared by reversing the order of putting the substrate solution and the precipitation reagent. An activity value was calculated by the following calculation formula.

U/mL = ΔOD × F × dilution ratio (F is a conversion factor obtained from a tyrosine calibration curve)

(Method for measuring carboxymethyl cellulase activity)

A method for measuring a carboxymethyl cellulase activity is, for example, as follows. The amount of reducing sugar generated from a substrate sodium carboxymethylcellulose (CMC) was measured by a dinitrosalicylic acid (DNS) method.

For activity measurement, 0.5 mL of a sample solution (enzyme-containing solution) diluted with a 50 mM citrate buffer (pH 4.8) is put in a test tube, 0.5 mL of a substrate solution is added thereto and mixed, and the mixture is caused to react at 50°C for 30 minutes. 3 mL of a DNS test solution was added thereto and mixed, and the mixture was allowed to stand in a boiling water bath for five minutes, and then cooled in an ice-water bath. 16 mL of distilled water was added thereto and mixed, and an absorbance thereof was measured at 550 nm. A blank was prepared by replacing the sample solution with a citrate buffer. For a calibration curve, 1 mL of a glucose standard solution (0 mg/mL, 0.2 mg/mL, or 0.5 mg/mL) was dispensed into a test tube, 3 mL of a DNS test solution was added thereto and mixed, and the mixture was allowed to stand in a boiling water bath for five minutes and then cooled in an ice-water bath. 16 mL of distilled water is added thereto and mixed, an absorbance thereof is measured at 550 nm to create a calibration curve, and the amount of glucose is calculated. An activity value was calculated by the following calculation formula.

$$U/mL = \text{amount of glucose (mg)} \times 1000/(180 \times 30)/0.5 \times \text{dilution ratio}$$

[Reference Example 1: Production using commercial strain]

According to a conventional method, Aspergillus oryzae RIB40 was grown on a 4% malt extract (manufactured by Oriental Yeast Co., Ltd.) agar medium. In a 500 mL Erlenmeyer flask containing 100 mL of Czapek Dox Broth (manufactured by Difco), 5 mm square of a grown hyphae tip part was inoculated and cultured at 30°C for seven days to obtain a culture solution of Reference Example 1.

[Example 1]

<Purification method for reducing amount of contaminant enzymes>

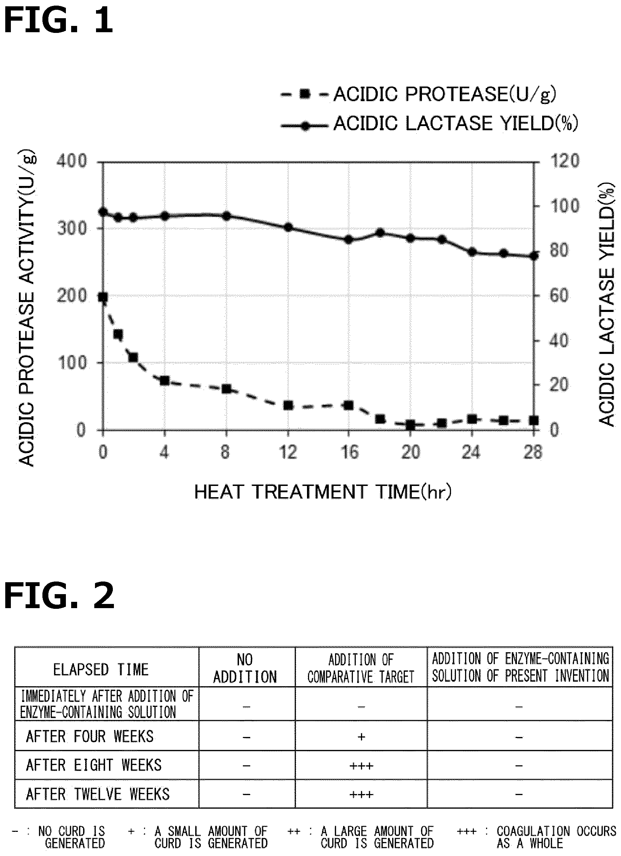

The culture medium of Reference Example 1 was adjusted to pH 6.5 using a sodium hydroxide solution, and then heat-treated at 50°C for 28 hours. Acidic protease could be removed while an acidic lactase activity was maintained (Fig. 1). Subsequently, the pH was adjusted to 5.2 using hydrochloric acid, and the treated culture solution was filtered using diatomaceous earth and a cellulose filtration aid to remove produced bacterial cells. Ammonium sulfate was added to the filtrate such that the filtrate was 55% saturated, and the mixture was stirred for three hours. Activated carbon was added thereto in an amount of 1.8% by volume of the ammonium sulfate treatment liquid, and the mixture was stirred for one hour. The ammonium sulfate activated carbon treatment liquid was filtered using diatomaceous earth and a cellulose filtration aid to remove a precipitate. The filtrate was desalted and concentrated with an ultrafiltration membrane at a pH of 5.5. The concentrated solution was filtered with a diatomaceous earth aid, 42% by mass of glycerin was added to the filtrate, and the mixture was well mixed. The mixed solution was sterilized with a filter to obtain an enzyme-containing solution. The obtained enzyme-containing solution had a ratio of the acidic protease to the acidic lactase of 0.004, an acidic lactase activity of 10813 U/g, an acidic protease activity of 33 U/g, and a carboxymethyl cellulase activity of 0.22 U/g.

[Comparative Example 1]

An enzyme-containing solution of Comparative Example 1 was obtained (acidic lactase activity: 10724 U/g, acidic protease activity: 230 U/g, carboxymethyl cellulase activity: 0.37 U/g) in a similar manner to Example 1 except that the step of performing heat treatment at 50°C for 28 hours was not performed among the steps of obtaining the enzyme-containing solution of Example 1.

<Evaluation 1: addition to raw material milk>

As the raw material milk, an acidic milk beverage containing a 35% milk component (30% skim milk and 5% milk protein powder), 40% fruit juice, a polysaccharide, and a stabilizer (pectin and gellan gum) and having pH of 4.1 was used (casein in the raw material milk: 11%). To 9 mL of the raw material milk, each of 0.1% by volume of the enzyme-containing solution of Example 1 and 0.1% by volume of the enzyme-containing solution of Comparative Example 1, which were obtained as described above, was added at an acidic lactase activity of 1500 U/mL, stirred, and allowed to stand at 33°C for twelve weeks. Final concentrations of the acidic lactase and the acidic protease in each of the dairy products are presented in Table 1. In a case of using the enzyme-containing solution of Comparative Example 1, an aggregate was confirmed after four weeks, and the entire milk became curd and some areas of water separation was observed after eight weeks. On the other hand, in the enzyme-containing solution of Example 1 in which the amount of contaminant enzymes was reduced, no coagulation was observed at all even after twelve weeks as in a case of a system without addition of the enzyme-containing solution, and a smooth liquid was maintained (Fig. 2).

[Table 1]

|  | EXAMPLE 1 | COMPARATIVE EXAMPLE 1 |
|---|---|---|
| ACIDIC LACTASE (U/L) | 1500 | 1500 |
| ACIDIC PROTEASE (U/L) | 4.6 | 32.1 |

<Evaluation 2: addition to raw material milk>

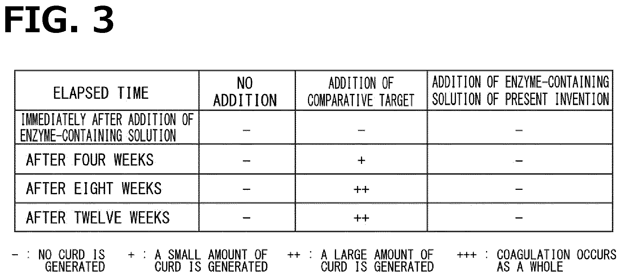

As the raw material milk, an acidic milk beverage containing a 25% milk component (25% skim milk), 30% fruit juice, a polysaccharide, and a stabilizer (pectin and gellan gum) and having pH of 4.5 was used (casein in the raw material milk: 6%). To 9 mL of the raw material milk, each of 0.1% by volume of the enzyme-containing solution of Example 1 and 0.1% by volume of the enzyme-containing solution of Comparative Example 1, which were obtained as described above, was added at an acidic lactase activity of 1500 U/mL, stirred, and allowed to stand at 33°C for twelve weeks. Final concentrations of the acidic lactase and the acidic protease in each of the dairy products are the same as those presented in Table 1. In a case of using the enzyme-containing solution of Comparative Example 1, an aggregate was confirmed after four weeks, the amount of the aggregate increased after eight weeks, and a similar result was observed after twelve weeks. On the other hand, in the enzyme-containing solution in which the amount of contaminant enzymes was reduced, no coagulation was observed at all even after twelve weeks as in a case of a system without addition of the enzyme-containing solution, and a smooth liquid was maintained (Fig. 3).

**EP 4 495 233 A1**

**Claims**

1. An enzyme-containing solution comprising acidic lactase and having an acidic protease activity of 100 U/g or less.

2. The enzyme-containing solution according to claim 1, wherein a ratio of the acidic protease activity (U/g) to the acidic lactase activity (U/g) is 0.01 or less.

3. The enzyme-containing solution according to claim 1 or 2, wherein a ratio of a carboxymethyl cellulase activity (U/g) to the acidic lactase activity (U/g) is $0.3 \times 10^{-4}$ or less.

4. A method for producing a dairy product, comprising a step of adding an enzyme-containing solution to a raw material milk containing casein, wherein
   the enzyme-containing solution contains acidic lactase and has an acidic protease activity of 100 U/g or less.

5. The method for producing a dairy product according to claim 4, wherein a content of the casein in a raw material milk is 3 to 20% by mass.

6. The method for producing a dairy product according to claim 4 or 5, wherein the raw material milk is an acidic milk.

7. The method for producing a dairy product according to any one of claims 4 to 6, wherein the acidic protease activity to the casein is 300 mU/g or less.

# FIG. 1

Legend: - ■ - ACIDIC PROTEASE(U/g)  —◆— ACIDIC LACTASE YIELD(%)

Y-axis (left): ACIDIC PROTEASE ACTIVITY(U/g) — 0, 100, 200, 300, 400
Y-axis (right): ACIDIC LACTASE YIELD(%) — 0, 20, 40, 60, 80, 100, 120
X-axis: HEAT TREATMENT TIME(hr) — 0, 4, 8, 12, 16, 20, 24, 28

# FIG. 2

| ELAPSED TIME | NO ADDITION | ADDITION OF COMPARATIVE TARGET | ADDITION OF ENZYME-CONTAINING SOLUTION OF PRESENT INVENTION |
|---|---|---|---|
| IMMEDIATELY AFTER ADDITION OF ENZYME-CONTAINING SOLUTION | − | − | − |
| AFTER FOUR WEEKS | − | + | − |
| AFTER EIGHT WEEKS | − | +++ | − |
| AFTER TWELVE WEEKS | − | +++ | − |

− : NO CURD IS GENERATED   + : A SMALL AMOUNT OF CURD IS GENERATED   ++ : A LARGE AMOUNT OF CURD IS GENERATED   +++ : COAGULATION OCCURS AS A WHOLE

# FIG. 3

| ELAPSED TIME | NO ADDITION | ADDITION OF COMPARATIVE TARGET | ADDITION OF ENZYME-CONTAINING SOLUTION OF PRESENT INVENTION |
|---|---|---|---|
| IMMEDIATELY AFTER ADDITION OF ENZYME-CONTAINING SOLUTION | − | − | − |
| AFTER FOUR WEEKS | − | + | − |
| AFTER EIGHT WEEKS | − | ++ | − |
| AFTER TWELVE WEEKS | − | ++ | − |

− : NO CURD IS GENERATED    + : A SMALL AMOUNT OF CURD IS GENERATED    ++ : A LARGE AMOUNT OF CURD IS GENERATED    +++ : COAGULATION OCCURS AS A WHOLE

## INTERNATIONAL SEARCH REPORT

| International application No. |
|---|
| **PCT/JP2023/010684** |

**A. CLASSIFICATION OF SUBJECT MATTER**

*C12N 9/38*(2006.01)i; *A23C 9/152*(2006.01)i; *C12N 9/58*(2006.01)i; *C12P 1/00*(2006.01)i
FI: C12N9/38; A23C9/152; C12N9/58; C12P1/00 Z

According to International Patent Classification (IPC) or to both national classification and IPC

**B. FIELDS SEARCHED**

Minimum documentation searched (classification system followed by classification symbols)

C12N9/38; A23C9/152; C12N9/58; C12P1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2022-504684 A (CHR. HANSEN A/S) 13 January 2022 (2022-01-13) claims, examples | 1-7 |
| X | WO 2013/168438 A1 (INCORPORATED NATIONAL UNIV. IWATE UNIVERSITY) 14 November 2013 (2013-11-14) claims, examples, paragraph [0066] | 1-7 |
| X | CN 101768578 A (FEED RESEARCH INSTITUTE, CHINESE ACADEMY OF AGRICULTURAL SCIENCES) 07 July 2010 (2010-07-07) claims, examples, paragraph [0026] | 1-7 |
| X | CN 112646795 A (ZHONGNUO BIOTECHNOLOGY DEVELOPMENT JIANGSU CO., LTD.) 13 April 2021 (2021-04-13) claims, examples, paragraph [0002] | 1-7 |

☐ Further documents are listed in the continuation of Box C.   ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **28 April 2023** | **16 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/010684**

| Patent document cited in search report | | | Publication date (day/month/year) | Patent family member(s) | | | Publication date (day/month/year) |
|---|---|---|---|---|---|---|---|
| JP | 2022-504684 | A | 13 January 2022 | US claims, examples CN | 2021/0348147 112654251 | A1 A | |
| WO | 2013/168438 | A1 | 14 November 2013 | (Family: none) | | | |
| CN | 101768578 | A | 07 July 2010 | (Family: none) | | | |
| CN | 112646795 | A | 13 April 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Milk Science*, 2011, vol. 60 (2)
- *Milk Science*, 2012, vol. 61 (3)

- United States Food Chemicals Codex (FCC). 01 July 1996, 802-803